# EUROPEAN PATENT APPLICATION

(11) **EP 2 937 682 A1**
(43) Date of publication of application: **28.10.2015**
(21) Application number: 14166012.6
(22) Date of filing: 25.04.2014
(51) Int. Cl.: G01N 15/14, A61B 3/10, A61B 5/00, G01N 21/47, G01N 15/10

(54) **Method and apparatus for optically differentiating objects**

(71) Applicant: PZ Cormay S.A., 05-092 Lomianki (PL)
(72) Inventor: Szkulmowska, Anna, 87-100 Torun (PL); Wojtkowski, Maciej, 87-100 Torun (PL)
(74) Representative: Glawe, Delfs, Moll

(57) **Abstract**

The invention relates to a method and an apparatus (1) for optically differentiating objects.

The inventive method comprises the steps of:
- Illuminating a scattering medium with a light beam (30) from a light source (3), wherein an object (10) to be differentiated moves in the light beam (30) so that light modulated by the object (10) hits the scattering medium;
- Registering the light scattered by the scattering medium with low-coherence interferometry (4); and
- Analyzing the registered scattered light for patterns in intensity and/or phase changes caused by the moving object (10) in order to differentiate the object (10).

The inventive apparatus (1) comprises a light source (3), a scattering base (2) comprising a scattering medium, a low-coherence interferometry detection device (4) and an evaluation unit (5), wherein the light source (3) is configured to illuminate the scattering base (2), the low-coherence interferometry detection device (4) is configured to detect the light scattered by the scattering base (2), and an object path (90) that is non-parallel to the light beam (31) of the light source (3) is arranged between the light source (3) and the scattering base (2), wherein the evaluation unit (5) is configured to analyze the changes in intensity and/or phase in the light scattered by the scattering base (2) and detected by the detection device (4) for patterns that are caused by an object (10) moving along the object path (90).

## Description

The present invention relates to a method and an apparatus for optically differentiating objects.

In the state of the art, there are a number of different methods known to differentiate small objects, e.g. cells or bacteria. In some of these methods light is used to illuminate a small object to be differentiated. The light that hits the small object to be differentiated is scattered in a specific way depending on the type of the small object. The light scattered by the small object is collected by a detection unit and can be analyzed to differentiate the small object.

Due to the small size of the object to be differentiated - which may be in the range of a few microns - the quantity of light scattered by the object and consequently the information bandwidth provided by the detection unit is limited. Actual differentiation of small objects is thus difficult and subject to certain inaccuracies.

It is an object of the present invention to provide a method and an apparatus to differentiate small objects that are improved over the mentioned state of the art.

This objective is achieved by the method according to claim 1 as well as the apparatus according to claim 8. Preferred further improvements are subject matter of the dependent claims.

According to this, the invention relates to a method to optically differentiate objects comprising the steps of
- Illuminating a scattering medium with a light beam from a light source, wherein an object to be differentiated moves in the light beam so that light modulated by the object hits the scattering medium;
- Registering the light scattered by the scattering medium with low-coherence interferometry; and
- Analyzing the registered scattered light for patterns in intensity and/or phase changes caused by the moving object in order to differentiate the object.

The invention also relates to an apparatus comprising a light source, a scattering base comprising a scattering medium, a low-coherence interferometry detection device and an evaluation unit, wherein the light source is configured to illuminate the scattering base, the low-coherence interferometry detection device is configured to detect the light scattered by the scattering base, and an object path that is non-parallel to the light beam of the light source is arranged between the light source and the scattering base, wherein the evaluation unit is configured to analyze the changes in intensity and/or phase in the light scattered by the scattering base and detected by the detection device for patterns that are caused by an object moving along the object path.

Before discussing the invention in detail, a few terms used in connection therewith are explained:
The term "scattering medium" refers to a medium that scatters light that hits and at least partially penetrates the medium, wherein light rays are deflected from their usual straight path. The scattering medium may scatter the light e.g. by irregularities or particles within or on the surface of the scattering medium.

"To move in a light beam" in context with the present invention defines a movement of an object that is non-parallel, e.g. orthogonal, to the axis of the light beam during with the object is constantly illuminated by the light of the light beam. For the object to be differentiated to move in the light beam, the object itself might move and/or - especially in case the object is stationary - the light source and/or the scattering medium is moved relative to the object.

The term "micron sized object" used in the following relates to an object whose characteristic dimension is smaller than 1 mm, preferably smaller than 0,5 mm, preferably smaller than 0,1 mm. The characteristic dimension of an object may, for example, be its diameter or its length.

With the method according to the invention, the signal used to differentiate the object does not originate from the object itself but rather results from modifications in the illuminated scattering medium caused by the modulation of the light by the object to be differentiated. It has been found that different objects cause different modifications of the light coming from the scattering medium. Analyzing the light received from the scattering medium allows conclusions on the characteristics of the object and thus a differentiation. Since the quantity of light receivable from the scattering medium that bears information on the object in form of modulations is greater that the quantity of light that can usually be received directly from the object according to the state of the art, the scattering medium can be seen as an amplifier for the light modulated by the object.

In the claimed method, a scattering medium is illuminated by a light beam of a light source. Due to its characteristics, said medium scatters the light. The actual scattering by the scattering medium might be random. In any case, it is preferred that the scattering pattern of the scattering medium is constant over time, i.e. the characteristics, especially the scattering pattern of said medium does not change during an investigation of one or more objects.

The light scattered by the scattering medium is registered with low-coherence interferometry. In case no object to be differentiated is present, the registered scattered light represents the scattering pattern of the scattering base, which, in case of a constant scattering pattern, also remains constant.

According to the invention, an object is moved in the light beam so that light modulated by the object hits the scattering medium. In other words: the object to be differentiated is generally located in the light beam between the light source and the scattering medium. The light from the light source crossing the object is modulated by the object and hits the scattering medium after this modulation. Since the object to be differentiated is moved in the light beam according to the invention, the modulated light coming from the object causes a change in intensity and/or phase of the light scattered by the scattering medium. These changes can be registered with the low-coherence interferometry.

When analyzing the registered light coming from the scattering medium, it has been found that different types of objects moved in the light beam result in distinct patterns of changes in intensity and/or phase of said light. Therefore, preferably using statistical methods, patterns can be defined for a plurality of different types of objects. For example, these patterns may result from an analysis of the registered changes in intensity and/or phase of the light scattered by the scattering medium caused by objects whose type is known, e.g. a known micron sized object or a specific cell.

Said patterns may then be used to differentiate objects whose type is unknown. The changes of intensity and/or phase caused by an object whose type is unknown may be matched with previously determined patterns. If the changes caused by an investigated object match a previously determined pattern to a predetermined degree, the investigated object may be regarded as being of the same type used to determine the pattern.

The changes in intensity and/or phase of the light coming from the scattering medium only appears if said medium indeed scatters the light impinging on it and the object to be differentiated is indeed moved in the light beam.

The movement of the object to be differentiated may occur autonomously from conducting the inventive method or may be caused by using a suitable device. The first mentioned alternative may, for example, occur in already existing or natural systems, while the latter alternative is generally found in artificial systems.

In already existing or natural systems, the scattering medium and/or the object to be differentiated might be elements occurring and situated in pre-existing or naturally occurring structure. The object to be differentiated might for example be a blood cell flowing through a conduit, e.g. a vessel, while the scattering medium might be tissue, e.g. retinal tissue, surrounding said conduit. The movement of the object to be differentiated might be caused by mechanisms detached from the inventive method, e.g. a naturally occurring circulation. If a light beam is directed on the scattering medium in the area of said conduit, the object flowing through said conduit moves in the light beam within the sense of the present invention, thus causing changes in intensity and/or phase of the light after being scattered by the scattering medium. As explained above, the light coming from the scattering medium may be registered and analyzed to differentiate the object causing said changes in intensity and/or phase.

In artificial systems, the object to be differentiated may be synthesized and/or a sample isolated from other systems in advance to conducting the inventive method, and may be moved in the light of a light source according to the invention by a suitable actuation device. In case the object to be differentiated is introduced in a solution, the object may be moved though a channel, a tube or a pipe by means of a pumping device producing a flow of the solution carrying the object within the channel, tube or pipe. The channel, tube or pipe is preferably transparent. For example, the object might be a blood cell in a preferably diluted blood sample obtained separately from the inventive method, wherein the blood sample flows through a microchannel, i.e. a channel with a diameter comparable to the micron size of a single blood cell. Alternatively, the object might be moved by an air flow carrying the object along or by applying an (electro-)magnetic field that exerts a force on the object to be differentiated.

The scattering medium may also be manufactured and/or previously isolated from other systems in advance to conducting the inventive method. For example, the scattering medium may comprise or consist of paper, a fat emulsion solution, a polymer solution and/or a polymer body. Preferably, the scattering medium comprises a body made of polymer mixed with a pigment, preferably titanium white. The light coming from the scattering medium of an artificial system may be registered and analyzed to differentiate the object causing said changes in intensity and/or phase in accordance with the invention.

Of course, all hybrid forms of the provided examples of natural and artificial systems are possible.

As indicated above, the movement of the object to be differentiated may be achieved by the object moving in relation to a stationary light source and a stationary scattering medium. Alternatively, the object to be differentiated may remain stationary, while the light source and scattering medium are moved in relation to the object.

Preferably, the low-coherence interferometry for registering the light scattered by the scattering medium may be optical coherence tomography.

Preferably, the object to be differentiated is a micron sized object, which might be of biological or any other origin, preferably a cell or a bacterium.

The analysis of the scattered light preferably provides information on size, shape and/or internal structure of the object to be differentiated. Corresponding information may be obtained by statistically analyzing known object with the inventive method where - after sufficient samples have been analyzed - said information on unknown objects may be obtained by comparing the changes in intensity and/or phase caused be said unknown object to the results obtained using the known objects.

The apparatus according to the invention comprises a light source, a scattering base comprising a scattering medium, a low-coherence interferometry detection device and an evaluation unit, wherein the light source is configured to illuminate the scattering base, the low-coherence interferometry detection device is configured to detect the light scattered by the scattering base, and an object path that is non-parallel to the light beam of the light source is arranged between the light source and the scattering base, wherein the evaluation unit is configured to analyze the changes in intensity and/or phase in the light scattered by the scattering base and detected by the detection device for patterns that are caused by an object moving along the object path.

The object path may be defined by a channel provided in the scattering base, or a tube or a pipe arranged between the light source and the scattering base through which the object to be differentiated moves. Preferably, the diameter of the channel, tube or pipe may be adapted to the size of the objects to be differentiated, e.g. a channel might be dimensioned to allow the passing of a single object at a time, e.g. a cell. Furthermore, the channel, tube or pipe is preferably transparent.

The apparatus preferably comprises an actuation device for moving an object along the object path. Depending on the configuration of the object path, the properties of the object to be differentiated and whether the object floats in a solution, this actuation device might be a pumping device, a fan or a device for generating (electro-)magnetic fields.

The scattering base may comprise or consist of paper, a fat emulsion solution, a polymer solution and/or a polymer body as the scattering medium. Preferably, the scattering base comprises a body made of polymer mixed with a pigment, preferably titanium white.
Preferably, the light source is a component of the low-coherence interferometry detection device. The low-coherence interferometry detection device may be an optical coherence tomography device, preferably comprising the light source.

For further explanation of the inventive apparatus it is referred to the remarks regarding the inventive method.

The invention will now be described in more detail based on exemplary embodiments with reference to the enclosed figures. These figures show:
- Figure 1:: a first embodiment of an apparatus according to the invention;
- Figure 2a, b:: schematic representations of exemplary results achieved with the apparatus according to figure 1;
- Figure 3a, b:: a set of measurement result achieved with an apparatus according to figure 1; and
- Figure 4a, b:: another set of measurement results achieved by conducting the inventive method.

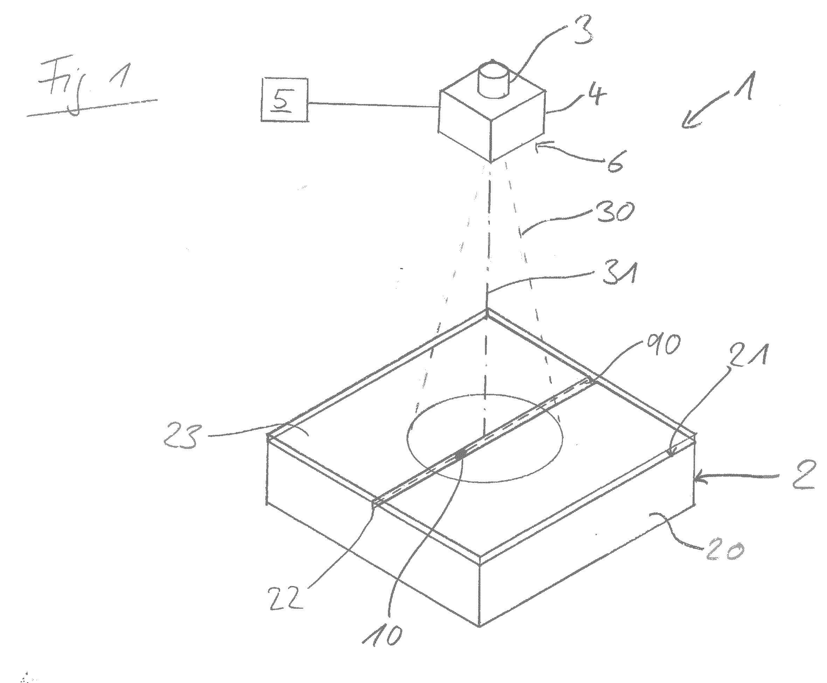

Figure 1 shows a first embodiment of the apparatus 1 according to the invention. By means of explaining the apparatus 1, the inventive method will become readily apparent.

The apparatus 1 comprises a scattering base 2, a light source 3, a low-coherence interferometry detection device 4 and an evaluation unit 5.

The scattering base 2 comprises a scattering body 20 made of scattering medium, in this example a polymer mixed with titanium white. On the top surface 21 of the scattering body 20, a channel 22 with a depth of approx. 40 microns and a width of approx. 100 microns is etched to define an object path 90. To seal the channel 22, a transparent glass slide 23 is affixed on the top surface 21 of the scattering body 20.

The light source 3 and the low-coherence interferometry detection device 4 are both parts of a single device, i.e. an optical coherence tomography device 6. The light source 3 illuminates the scattering base 2 in a way that the object path 90 traverses the light beam 30 created by the light source 3. The object path 90 runs orthogonally to the axis 31 of the light beam 30. The light from the light source 3-after having passed through the glass slide 23 - is scattered by the scattering body 20. The light scattered by the scattering body 20 is registered by the low-coherence interferometry detection device 4.

The low-coherence interferometry detection device 4 is connected to the evaluation unit 5 that is configured to analyze the measured data provided by the detection device 4.

In order to differentiate an object 10, the object 10 according to the invention needs to move along the object path 90 in the light beam 30. In order to achieve said movement, the object 10 is situated in the channel 22 that is otherwise filled a fluid, in the present case a transparent fluid. The fluid might be set in motion by a pump (not shown) that either circulates the fluid around a fluid path, part of which is constituted by the channel 22 or moves the fluid from a reservoir (not shown) via the channel 22 to a collection container (not shown). The object 10 is then dragged along with the fluid stream and thus moved.

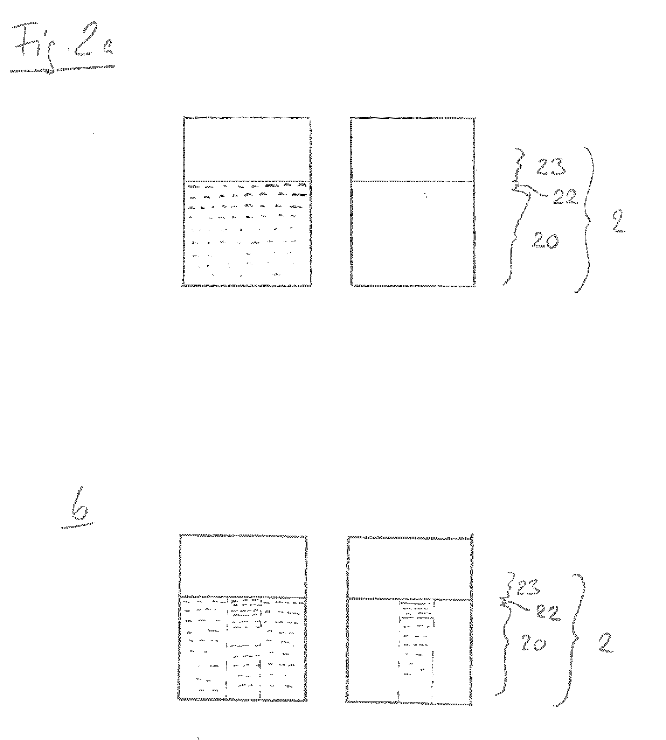

In Figures 2a, b, schematic graphical representations of a different set of measured data provided by the detection device 4 as prepared by the evaluation unit 5 is illustrated. On the left in each Figure 2a and 2b, the intensity of the light scattered by the scattering base 2 along the object path 90 is shown, while the graph on the right in each Figure 2a and 2b shown the phase change of the light scattered by the scattering base 2 along the object path 90. Due to its transparency, the glass slide 23 does not appear in the graphs. The scattering only occurs in the scattering body 20.

Figure 2a shows the measured data in case either no object 10 is present in the channel 22 within the light beam 30 or an object 10 is indeed present in said region but is not moving. In this case, the intensity of the light scattered by the scattering base 2 or rather the scattering body 20 is - in the present example - substantially uniform along the object path 90, decreasing with the depth of penetration of the scattering body 20. Due to the material of the scattering body 20, the scattering of the light in the scattering body 20 remains constant over time.

In case no object 10 is present in the channel 22 within the light beam 30 or an object 10 is indeed present in said region but is not moving, no phase change of the light scattered by the scattering base 2 can be detected. Therefore the graph on the right of figure 2a is empty.

Figure 2b shows the situation, in case an object 10 to be differentiated is moving in the light beam 30 along the object path 90. In this case, changes in intensity and phase of the light scattered by the scattering base 2 can be observed. These changes are caused by the light being modulated by the object 10.

It has been found that the changes in intensity and phase of the light scattered by the scattering base 2 differ depending on the type of object 10 causing said changes. Therefore, these changes detected by the detection device 4 can be processed by the evaluation unit 5 to differentiate objects 10 moving along the object path 90.

The actual differentiation of objects 10 and possible identification of their type may be done statistically. For example, intensity and phase changes of objects 10, whose types are knows, may be examined with the apparatus 1 for patterns. Consecutively examined objects 10, whose types are unknown, might be differentiated by comparing the changes in intensity and phase of the light scattered by the scattering base 2 cause by the objects 10 to the previously determined pattern. This statistical analysis can be achieved by the evaluation unit 5.

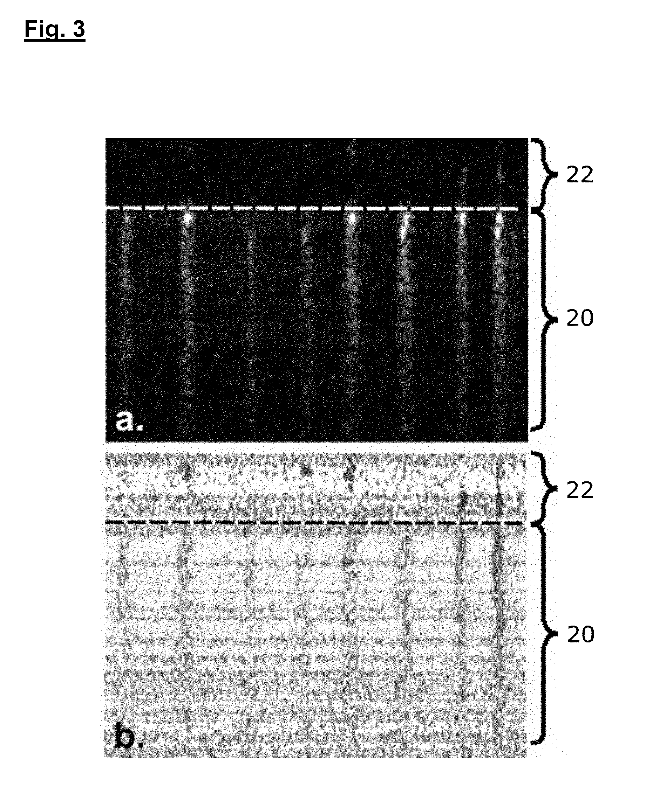

Figures 3a, b shows an actual measurement conducted with an apparatus 1 according to figure 1. In this example, several objects 10 are moving in the channel 22 and cause detectable changes in intensity (Figure 3a) and phase (Figure 3b) of the light scattered by the scattering body 20. In this example, the eight objects 10 moving along the object path 90 as defined by the channel 22 within the light beam 30 can be clearly localized by the changes that can be observed in the scattering body 20, while the objects 10 in the channel 22 themselves are hardly visible. The changes in intensity and phase of the light scattered by the scattering base 2 caused by each object 10 individually are also suitable to differentiate each of the objects 10.

The objects 10 to be differentiated as shown in figures 3a, b may be blood cells from a sample obtained and possibly diluted in advance to analyzing it according to the invention. The objects 10 might also be synthesized microparticles resulting from a chemical or biochemical process.

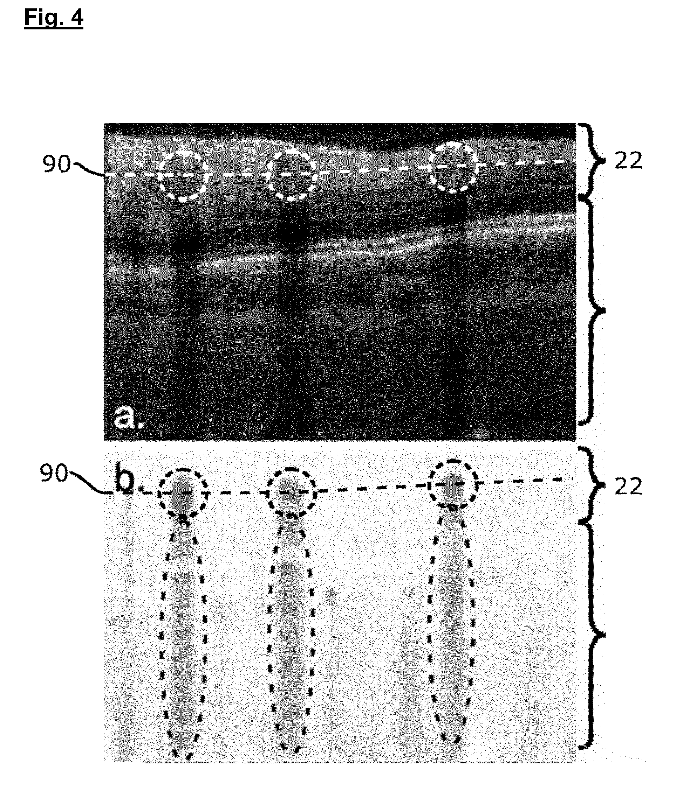

Figures 4a, b show actual measurements conducted in accordance with the inventive method. The objects 10 to be differentiated are blood cells. However, instead of a manufactures scattering base 2, the blood cells are moving through vessels defining the object path 90, while the tissue surrounding said vessel serves as the scattering medium. The vessel and the surrounding tissue may be located on the retina of an eye of a living creature and thus accessible by the inventive method without any contact or any interference with the body of the living creature. The vessel may be compared to the channel 22, the tissue to the scattering body 20. The other components of the apparatus 1 as shown in figure 1 as well as the described procedure to differentiate an object 10 remain unaltered.

In Figure 4a, b three objects 10 are clearly identifiable and - due to the changes in intensity (Figure 4a) and phase (Figure 4b) of the light scattered by the scattering body 20 - can be differentiated.

## Claims

1. Method to optically differentiate objects (10) comprising the steps of:
- Illuminating a scattering medium with a light beam (30) from a light source (3), wherein an object (10) to be differentiated moves in the light beam (30) so that light modulated by the object (10) hits the scattering medium;
- Registering the light scattered by the scattering medium with low-coherence interferometry; and
- Analyzing the registered scattered light for patterns in intensity and/or phase changes caused by the moving object (10) in order to differentiate the object (10).

2. Method according to claim 1, wherein
the object (10) is moved autonomously from conducting the method or is moved by an actuation device.

3. Method according to any one of the preceding claims,
wherein
the object (10) moves in relation to a stationary light source (3) and a stationary scattering medium, or the object (10) is stationary and the light source (3) and the scattering medium move in relation to the object.

4. Method according to any one of the preceding claims,
wherein
the low-coherence interferometry is optical coherence tomography.

5. Method according to any one of the preceding claims,
wherein
the object (10) to be differentiated is a micron sized object of biological or any other origin, preferably a cell or a bacterium.

6. Method according to any one of the preceding claims,
wherein
the analysis of the scattered light provides information on size, shape and/or internal structure of the object (10) to be differentiated.

7. Method according to any one of the preceding claims,
wherein
the scattering medium comprises or consists of paper, a fat emulsion solution, a polymer solution and/or a polymer body, preferably a body made of polymer mixed with a pigment, preferably titanium white.

8. Apparatus (1) comprising a light source (3), a scattering base (2) comprising a scattering medium, a low-coherence interferometry detection device (4) and an evaluation unit (5), wherein the light source (3) is configured to illuminate the scattering base (2), the low-coherence interferometry detection device (4) is configured to detect the light scattered by the scattering base (2), and an object path (90) that is non-parallel to the light beam (31) of the light source (3) is arranged between the light source (3) and the scattering base (2), wherein the evaluation unit (5) is configured to analyze the changes in intensity and/or phase in the light scattered by the scattering base (2) and detected by the detection device (4) for patterns that are caused by an object (10) moving along the object path (90).

9. Apparatus according to claim 8, wherein
object path (90) is defined by a channel (22) provided in the scattering base (2), or by a tube or a pipe arranged between the light source (3) and the scattering base (2), wherein the channel (22), tube or pipe is preferably transparent.

10. Apparatus according to claim 9, wherein
the diameter of the channel (22), tube or pipe is adapted to the size of the objects (10) to be differentiated.

11. Apparatus according to any one of the claims 8-10,
wherein
the apparatus (1) comprises an actuation device for moving an object (10) along the object path (90).

12. Apparatus according to any one of the claims 8-11,
wherein
the scattering base (2) may comprise or consist of paper, a fat emulsion solution, a polymer solution and/or a polymer body as the scattering medium.

13. Apparatus according to any one of the claims 8-11,
wherein
the scattering base (2) comprises a scattering body (20) made of polymer mixed with a pigment, preferably titanium white.

14. Apparatus according to any one of the claims 8-13,
wherein
the light source (2) is a component of the low-coherence interferometry detection device (4).

15. Apparatus according to any one of the claims 8-14, wherein the low-coherence interferometry detection device (4) is an optical coherence tomography device.
